Europäisches Patentamt

European Patent Office

Office européen des brevets

(11) Publication number: **0 395 765**
**A1**

## EUROPEAN PATENT APPLICATION
### published in accordance with Art. 158(3) EPC

(21) Application number: **89901311.4**

(22) Date of filing: **11.01.89**

(86) International application number:
**PCT/JP89/00022**

(87) International publication number:
**WO 89/06532 (27.07.89 89/16)**

(51) Int. Cl.5: **A61K 9/10**

(30) Priority: **13.01.88 JP 5361/88**

(43) Date of publication of application:
**07.11.90 Bulletin 90/45**

(84) Designated Contracting States:
**AT BE CH DE FR GB IT LI LU NL SE**

(71) Applicant: **Dainippon Pharmaceutical Co., Ltd.**
**25, Doshomachi 3-chome Higashi-ku**
**Osaka-shi, Osaka 541(JP)**

(72) Inventor: **YASUI, Kiyotada**
**14-24, Kitahon-cho 2-chome**
**Yao-shi Osaka 581(JP)**
Inventor: **HIGASHI, Toshiro**
**7-11, Shioyakita-cho 3-chome Tarumi-ku**
**Kobe-shi Hyogo 655(JP)**
Inventor: **IMASATO, Yuu**
**5-A3-202, Shinsenrihigashi-cho 2-chome**
**Toyonaka-shi Osaka 565(JP)**

(74) Representative: **Kraus, Walter, Dr. et al**
**Patentanwälte Kraus, Weisert & Partner**
**Thomas-Wimmer-Ring 15**
**D-8000 München 22(DE)**

(54) **OOZING-PROOF LIPOSOME PREPARATION.**

(57) A frozen or lyophilized liposome preparation containing a water-soluble high-molecular substance as the active ingredient, which contains gelatin, albumin or polyethylene glycol in the inner aqueous phase of liposome. This preparation does not cause oozing of the high-molecular substance upon freezing or lyophilization, thus being quite useful as a liposome preparation of such a substance.

- 1 -

SPECIFICATION

LEAKAGE FREE LIPOSOME PREPARATION

Technological Field

This invention relates to a liposome preparation whose effective components after freezing or lyophilization are prevented from leakage. More specifically, it relates to a liposome preparation for freezing or lyophilization containing water-soluble polymeric substances as effective components in which the inner aqueous phase contains gelatin, alubumin or polyethylene glycol (to be referred to as "the preparation of the invention").

The liposome is classified as having an onion-like multi-thin layer structure composed of a number of phospholipid double layers at a fixed distance from each other by an aqueous substance (to be referred to as a "multilayer liposome") and one having a single phospholipid double layer containing an aqueous substance (to be referred to as a "single layer liposome").

In the present specification the phase outside phospholipid by layer located on the outermost side of the multilayer liposome or the outside of the single liposome will be called an "external aqueous phase", and phases in each phospholipid in the multilayer liposome or phase inside of the single layer liposome will be called an "inner aqueous phase".

Since liposome can include any water-soluble substances, is prone to undergo phagocytosis by macrophage, and is prone to be taken up by the reticuloendothelial system such as the liver, spleen, marrow, etc., active work is being done on liposome as one means of chemotherapy by which a drug is efficiently migrated into the living body.

Liposome itself is stable when stored in a refrigerated state, but many of water-soluble substances are unstable in solution when even stored in the

- 2 -

refrigerated state. These substances should absolutely be stored in the frozen or or lyophilized state. It is known however that a water-soluble substance included in the inner aqueous phase leakages on freezing or lyophylization, and this is one cause which limits the practical utility of a preparation of liposome.

Background Technology

One prior means of preventing leakage is to include dimethyl sulfoxide, glycerol, alanine or glycine betaine in the external aqueous phase [J. Pharm. Pharmacol. 38, 259-263 (1986)]; to include polysaccharides, polyvinylpyrrolidone and mannitol into the external aqueous phase [Int. J. Pharm. 22, 299-310 (1984)]; and to include dextran, gum arabic, polyvinyl alcohol, polyvinylpyrrolidone, male bovine alubumin, and sucroses into the external aqueous phase (Japanese Patent Publication No. 21449/1886). Japanese Patent Official Announcement No. 500102/1987 discloses that saccharides such as trehalose, maltose, lactose, sucrose, glucose and dextran are used both in the inner and external aqueous phases. In any case, the effect of preventing leakage is insufficient.

Disclosure of the Invention

The present inventors found that by including gelatin, alubumin or polyethylene glycol into the inner aqueous phase of liposome, leakage of a water-soluble polymeric substance by freezing or lyophilization can be sufficiently prevented, and have thus completed the present invention.

Examples of water-soluble polymeric substances as effective ingredients which is included in the liposome of the preparation of the invention include tumor necrosis factor (to be abbreviated as "TNF"), interleukin I (to be abbreviated as "IL-1"), interferon, insulin, urokinase, prourokinase, hemoglobin, super oxide dismutase, somatostatin and heparin. They may be used

- 3 -

singly or in combination or in combination with a known stabilizer.

The liposome used in this invention may be any of the multilayer liposome and the single layer liposome.

Cholesterol may be used as a membrane reinforcing agent, and phosphatidic acid, dicetyl phosphate and stearylamine may be added as a charging substance.

One example of the outline of producing the preparation of the invention is described below.

An oleophilic substance is dissolved in an organic solvent, and in a nitrogen atmosphere or under vacuum, the solvent is removed by rotary evaporator. The lipid residue is formed into a film on the wall of a container. An aqueous solution of a water-soluble polymeric substance which is an effective ingredient, and gelatin, alubumin or polyethylene glycol in water is added, and the mixture is stirred or ultrasonicated to obtain a liposome dispersion. The dispersion may be filtered by filters having a decreasing opening size successively, or by a polycarbonate filter under a high pressure, or subjected to a high pressure emulsifier (MANTON-GAULIN emulsifier) to adjust the particle diameter. The resulting liposome dispersion is subjected to centrifugal separation, ultrafiltration, dialysis, gel filtration, etc. to separate and remove substances which are not taken up by the liposome, and the freezed, or lyophilized after addition of a saccharide such as mannitol or sucrose or amino acid such as glycine. In the case of freezing, it is preferred to add a known protective substance to the external aqueous phase.

The preparation of the invention produced as above, when frozen is thawed prior to use. The lyophilized preparation may be used either directly or by addition of a suitable aqueous solvent. Both may be used as an injectable, an orally administrable agent, or an externally applicable agent.

– 4 –

Brief Description of the Drawings

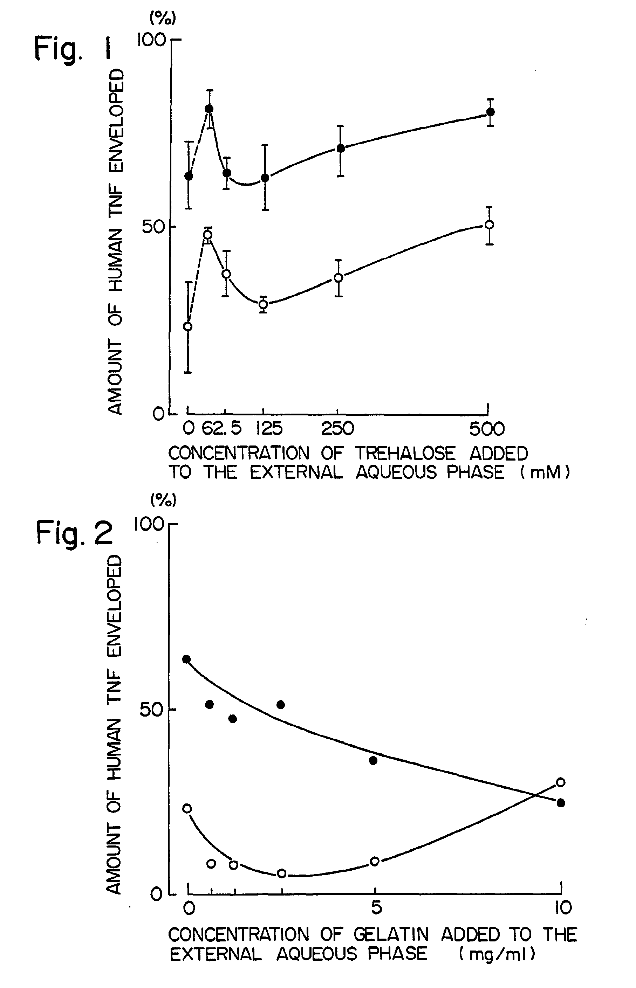

Figure 1 shows the effect of the concentration of trehalose added to the external aqueous phase upon the entrap ratio of included human TNF after thawing, and Figure 2 shows the effect of gelatin concentration added to the external aqueous phase. In these drawings, black circular marks represent the liposome preparation of the invention, while the white circular marks, represent control liposome preparation.

Best Mode of Practising the Invention

The following examples further illustrate the invention in detail, but they do not limit the present invention.

Example 1

(1) Yolk phosphatidyl choline (1 g) was dissolved in 10 ml of chloroform. After removing of chloroform by rotary evaporator, a 50 mM phosphate buffer (pH 7.0) to which 24,000,000 JRU of human TNF prepared in Example 3 of Japanese Laid-Open Patent Publication No. 232097/1985 (to be simply "human TNF") and 80 mg of gelatin (molecular weight: about 100,000) were added was added. Glassbeads were added, and the mixture was stirred to disperse the lipid. The dispersion was successively filtered with membrane filters having a pore diameter of 0.8 micrometer, 0.65 micrometer, 0.45 micrometer, 0.3 micrometer and 0.22 micrometer to adjust the particle diameter, and then gel-filtered by using a Sepharose 4B column (eluent: 50 mM phosphate buffer pH 7.0), and to remove the drug and gelatin in the external aqueous phase.

(2) A liposome suspension was prepared in the same way as in (1) except that gelatin was not added.

(3) To the liposome suspensions obtained in (1) and (2) was added trehalose or gelatin in the amounts shown in Figures 1 and 2, and the suspensions were freezed by storing in a refrigerator kept at –70 °C for 1 hour, and

- 5 -

then thawed at room temperature. Then by an enzyme immonoassay (to be referred to as EIA") in accordance with the competitive method, the amount of human TNF in the external aqueous phase was measured. On the other hand, the total amount of human TNF was measured after solubilizing the liposomes with Triton X100. The entrap ratio was then calculated.

The results are shown in Figures 1 and 2.

It is clearly shown in Figures 1 and 2 that the liposome of this invention in which human TNF and gelatin were coexistent apparently had a higher entrap ratio of human TNF than the liposome not containing gelatin in the inner aqueous layer did. It is also seen from Figure 1 that the addition of trehalose to the external aqueous phase further increased the entrap ratio.

On the other hand, as shown in Figure 2, the addition of gelatin to the external aqueous phase decreased the entrap ratio in the liposome of this invention, and even in the liposome containing no gelatin in the inner aqueous phase, if the concentration of gelatin in external aqueous phase was 0 to 3 mg/ml (practical concentration), the entrap ratio was decreased. It showed different properties from conventonal liposome protecting substances such as glycerol or saccharides.

Example 1-bis

The gelatin-included liposome suspension prepared in Example 1, (1) was stored at 4 $^{O}$C for 24 hours. Another same suspension was stored for 10 minutes in a constant-temperature vessel at 40 $^{O}$C to the gelatin in the inner aqueous layer. The samples were stored for 1 hour in a refrigerator at -70 $^{O}$C to freeze them. The frozen samples were thawed at room temperature, and the entrap ratio of human TNF was measured by EIA competitive method. The former had the entrap ratio 67.9 %, and the latter had the ratio 68.0 %. The protective effect thus

- 6 -

was the same for both whether or not gelatin in the inner aqueous phase solled or gelled.

Example 2

(1)    Yolk phoaphatidyl choline (700 mg), 200 mg of cholesterol, and 100 mg of stearylamine were each dissolved in chloroform and mixed.  After removing the solvent, human TNF 24,000,000 JRU and 80 mg of gelatin (molecular weight: about 7,000) were added, and the mixture was stirred.  The mixture was filtered through a membrane filter.  Each of the aqueous solutions shown in Tables 1 or 2 was added to the liposome suspension in a ratio of 1:1, and the mixture was freezed and thawed, or freeze-dried and redissolved.  The freeze-drying was carried out by preliminary freezing at -50 $^{o}$C, primary drying at -20 $^{o}$C, and secondary drying at 20 $^{o}$C under 0.1 torr or less.

(2)    A control liposome was obtained by the same procedure as above (1) except that gelatin was not added.

(3)    With regard to the liposome (1) and (2), the entrap ratio of human TNF was measured by EIA competitive method.  As shown in Tables 1 and 2, the liposome entrapping gelatin apparently had a higher entrap ratio of human TNF after freezing and thawing and the freeze-drying and redissolving than the liposome which did not contain gelatin.

- 7 -

Table 1: Effects of gelatin and saccharides
on human TNF leakage from a liposome
after freeze-drying and re-dissolving

| Type of liposome<br><br>Additive to the external aqueous phase | Liposome entrapping 0.8 % gelatin | Control liposome |
|---|---|---|
| 20 % trehalose | 98.4 | 55.5 |
| 20 % sucrose | 96.4 | 66.4 |
| 10 % lactose | 90.1 | 56.3 |

Table 2: Effects of gelatin and saccharides
on human TNF leakage from the liposome
after freezing and thawing

| Type of liposome<br><br>Additive to the external aqueous phase | Liposome entrapping 0.8 % gelatin | Control liposome |
|---|---|---|
| Buffer | 80.4 | 73.8 |
| 20 % trehalose | >97.6 | 90.6 |
| 10 % lactose | 92.7 | 71.9 |
| 20 % sucrose | >97.6 | 89.2 |
| 10 % fructose | 96.4 | 82.2 |
| 10 % glucose | 97.5 | 83.8 |
| 10 % mannitol | >97.6 | 87.3 |

- 8 -

Example 3

(1)      Yolk phosphatydyl choline (900 mg), 100 mg of sodium dipalmitoyl phosphatidylate were dissolved in 10 ml of chloroform.  Chloroform was removed by a rotary evaporator, and 50 mM phosphate buffer (pH 7.0) containing human TNF 24,000,000 JRU and 200 mg of gelatin (molecular weight: about 7,000) was added and the mixture was stirred together with glass beads to disperse the lipid.  The mixture was filtered under a high pressure by a polycarbonate membrane filter (0.2 micrometer) to adjust the particle diameter.  The mixture was then gel-filtered by using a Sephalose 4B column (eluent: 50 mM phosphate buffer pH 7.0).  The drug and the gelatin in the external aqueous phase were removed to prepare a liposome suspension.  To the liposome suspension was added a 40 % aqueous solution of sucrose in a ratio of 1:1.  The mixture was stored for 1 hour in a refrigerator at -70 $^{\circ}$C, and thawed at room temperature.

(2)      A contol liposome was obtained by the same procedure as in (1) above except that gelatin was not added.

(3)      With regard to the liposomes (1) and (2) above, the amount of human TNF in the external aqueous phase was measured by EIA competitive method.  On the other hand, the total amount of human TNF was measured after the liposome was solubilized by Triton X100.  The entrap ratio was measured.

As shown in Table 3, the liposome in which the human TNF and gelatin are coexisted in the inner aqueous phase apparently had a higher entrap ratio of human TNF than the liposome not containing gelatin, and the addition of sucrose to the external aquoeus phase showed an effect of improving the entrap ratio.

- 9 -

Table 3: Human TNF holding ratio of the liposome
after freezing and thawing

| Additive to the external aqueous phase | Type of liposome | Liposome entrapping 2 % gelatin | Control liposome |
|---|---|---|---|
| Buffer | | 69.5 | 13.2 |
| 40 % sucrose | | 96.0 | 69.9 |

Example 4

A liposome preparation was prepared in the same way as in Example 3 except that 200 mg of bovine serum albumin was used instead of 200 mg of gelatin, and the entrap ratio was measured.

As shown in Table 4, the liposome coexisting human TNF and albumin in the inner aqueous phase apparently had a higher entrap ratio of human TNF, and the addition of sucrose to the external aqueous phase had an effect of further improving the entrap ratio.

Table 4: Human TNF holding ratio of the liposome
after freezing and thawing

| Additive to the external aqueous phase | Type of liposome | Liposome entrapping 2 % albumin | Control liposome |
|---|---|---|---|
| Buffer | | 71.9 | 13.2 |
| 40 % sucrose | | 97.7 | 69.9 |

- 10 -

Example 5

A liposome preparation was prepared in the same way as in Example 3 except that 200 mg of polyethylene glycol (molecular weight: about 20,000) was used instead of 200 mg of gelatin. The liposome preparation was frozen and thawed. It was also freeze-dried under the same conditions as in Example 2. In the same way as in Example 3, the entrap ratio was measured. As shown in Tables 5 and 6, the liposome preparation entrapping polyethylene glycol apparently had a higher entrap ratio of human TNF after freezing and thawing and freeze-drying and redissolving than the liposome which did not contain polyethylene glycol.

Table 5: Human TNF holding ratio of the human
TNF after freezing and thawing

| Type of liposome<br><br>Additive to the external aqueous phase | Liposome entrapping 2 % polyethylene glycol | Control liposome |
|---|---|---|
| Buffer | 62.7 | 13.2 |
| 40 % sucrose | 92.6 | 69.9 |

Table 6: Human TNF holding ratio of the liposome
after freeze-drying and re-dissolving

| Type of liposome<br><br>Additive to the external aqueous phase | Liposome entrapping 2 % polyethylene glycol | Control liposome |
|---|---|---|
| 40 % sucrose | 73.2 | 38.1 |

- 11 -

Example 6

Yolk phosphatidyl choline (700 mg), 200 mg of cholesterol and 100 mg of stearylamine were each dissolved in chloroform, and mixed. After the solvent was removed, 8.4 mg of human IL-1 prepared in Example 2 of Japanese Laid-Open Patent Publication No. 27122/1986 (hereinafter simply "human IL-1") and 80 mg of gelatin (molecular weight: about 7000) was added, and the mixture was stirred and then filtered through a membrane filter. The saccharides shown in Tables 7 and 8 were each added in the amounts indicated, and the mixture was freezed and thawed or freeze-dried and re-dissolved. The freeze-drying was carried out by preliminary freezing at -50 $^{\circ}$C, primary drying at -20 $^{\circ}$C, and secondary drying at 20 $^{\circ}$C under 0.1 torr or less. The re-dissolving was carried out by using 50 mM phosphate buffer (pH 7.0). The amount of human IL-1 was measured by EIA competitive method. The entrap ratio was measured in the same way as in Example 1.

As shown in Tables 7 and 8, the liposome entrapping gelatin had a human IL-1 entrap ratio of more than 95 % after freezing and thawing and after freeze-drying and re-dissolving.

- 12 -

Table 7: Human IL-1 holding ratio of the liposome
after freezing and thawing

| Additive to the external aqueous phase | Liposome entrapping 0.8 % gelatin |
|---|---|
| 20 % trehalose | >95.0 |
| 10 % lactose | >95.0 |
| 20 % sucrose | >95.0 |

Table 8: Human IL-1 holding ratio of the liposome
after freeze-drying and re-dissolving

| Additive to the external aqueous phase | Liposome entrapping 0.8 % gelatin |
|---|---|
| 20 % trehalose | >95.0 |
| 10 % lactose | >95.0 |
| 20 % sucrose | >95.0 |

Example 7

(1)        Yolk phosphatidyl choline (700 mg), 200 mg of cholesterol and 100 mg of a decylamine were each dissolved in chloroform, and mixed. The solvent was removed, and 10 ml of human TNF 24,000,000 JRU in 1 ml of 0.9 % sodium chloride solution containing 5 mM phosphate buffer (to be referred to as "TNF alone") and 80 mg of gelatin (molecular weight: about 7,000) were added. The mixture was stirred and filtered through a membrane filter. A 40 % aqueous solution of sucrose was added to the liposome suspension in a ratio of 1:1, and the

- 13 -

mixture was freeze-dried by preliminary freezing at -50 $^{O}$C, primary drying at -20 $^{O}$C and secondary drying at 20 $^{O}$C under 0.1 torr or less. The freeze-dried mixture was again dissolved using 50 mM phosphate buffer (pH 7.0).

(2)         The liposome preparation obtained in (1) and the human TNF alone were intravenously injected to healthy ddY-strain male mice so that the dose reached 300,000 JRU/mouse, and the rate of survival was examined on the next day. With human TNF alone, all test animals died, whereas the test animals injected with the human TNF liposome preparation all survived.

Example 8

Meth A sarcoma cells (2 x 10$^5$) were intra-dermally transplanted in BALB/c-strain female mice (8 weeks old). On the 7th day, the human TNF liposome preparation (3,000 JRU/mouse) prepared in Example 7, (1) was administered once in the tumor. On the 24th day after the transplantation, the degrees of curing was examined. All mice completely cured. Mice whose tumor weight decreased to not more than 1 % were judged to be completely cured.

From Examples 7 and 8, it can be seen that the human TNF liposome preparation of this invention shows a decrease in toxicity in comparison with the human TNF alone, and that it has a curing effect of TNF.

Industrial Utilizability

The preparation of this invention can prevent leakage of the water-soluble polymeric substance by freezing or lyophilization, and is extremely useful as a liposome preparation of the substance.

- 14 -

What is claimed is:

1.        A frozon or lyophilized liposome preparation having a water-soluble polymeric substance as an active ingredient, characterized in that the inner aqueous phase of the liposome contains gelatin, albumin or polyethylene glycol.

2.        The frozen liposome preparation of claim 1 which contains a saccharide or an amino acid in the external aqueous phase.

3.        The liposome preparation of claim 1 in which the water-soluble polymeric substance is a protein.

4.        The liposome preparation of claim 1 in which the protein is a human tumor necrosis factor or inter-leukin 1.

EP 0 395 765 A1

Fig. 1

(%)

AMOUNT OF HUMAN TNF ENVELOPED

CONCENTRATION OF TREHALOSE ADDED
TO THE EXTERNAL AQUEOUS PHASE ( mM )

Fig. 2

(%)

AMOUNT OF HUMAN TNF ENVELOPED

CONCENTRATION OF GELATIN ADDED TO THE
EXTERNAL AQUEOUS PHASE ( mg/ml )

# INTERNATIONAL SEARCH REPORT

International Application No   PCT/JP89/00022

**I. CLASSIFICATION OF SUBJECT MATTER** (if several classification symbols apply, indicate all) [6]

According to International Patent Classification (IPC) or to both National Classification and IPC

Int.Cl$^4$   A61K9/10

**II. FIELDS SEARCHED**

| Minimum Documentation Searched [7] | |
|---|---|
| Classification System | Classification Symbols |
| IPC | A61K9/10, A61K37/00-37/04 |

Documentation Searched other than Minimum Documentation
to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | JP, B2, 61-21449 (Battelle Memorial Institute) 27 May 1986 (27. 05. 86) Claim 4 & DE, A, 2,834,308 & FR, A, 2,399,241 & US, A, 4,229,360 | 1-4 |
| Y | JP, A, 58-174330 (Asahi Chemical Industry Co., Ltd.) 13 October 1983 (13. 10. 83) Claim & EP, A, 91,258 & FR, A, 2,530,472 & DE, A, 3,373,628 & US, A, 4,447,355 | 1-4 |
| Y | EP, A2, 251,001 (Biotest Wolfram G.m.b.H.) 07 January 1988 (07. 01. 88) Claim 1 | 1-4 |
| E | JP, A, 64-9931 (Daiichi Seiyaku Co., Ltd.) 13 January 1989 (13. 01. 89) Pages 1 to 6 (Family: none) | 1-4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 24, 1989 (24. 03. 89) | April 10, 1989 (10. 04. 89) |
| International Searching Authority | Signature of Authorized Officer |
| Japanese Patent Office | |